# FASCICULE DE BREVET EUROPEEN

(11) **EP 0 797 461 B1**
(45) Date de publication et mention de la délivrance du brevet: **10.09.2003**
(21) Numéro de dépôt: 96931933.4
(22) Date de dépôt: 10.10.1996
(51) Int. Cl.: A61M 1/16, G01N 1/20

(54) **DISPOSITIF DE RECUEIL D'UN ECHANTILLON DE LIQUIDE DE DIALYSE USE**
VORRICHTUNG ZUR PROBENENTNAHME VON VERBRAUCHTEM DIALYSAT
DEVICE FOR COLLECTING A SAMPLE OF USED DIALYSIS FLUID

(30) Priorité: 12.10.1995 FR 9512187
(43) Date de publication de la demande: 01.10.1997
(73) Titulaire: GAMBRO HOSPAL (Schweiz) AG, 4001 Basel (CH)
(72) Inventeur: NUCCIO, Antonino, I-42100 Reggio Emilia (IT)
(74) Mandataire: Lejeune, Daniel
(86) Numéro de dépôt international: IB9601071
(87) Numéro de publication internationale: WO97013535

(56) Documents cités:
- EP-A- 0 621 046
- EP-A- 0 711 569
- WO-A-82/04127
- FR-A- 2 696 644

## Description

L'invention concerne un dispositif pour recueillir un échantillon de liquide usé représentatif de la totalité du liquide usé évacué à tout moment depuis le début d'une séance de traitement de sang par circulation extracorporelle.

L'invention trouve une application particulière dans le traitement des patients souffrant d'insuffisance rénale chronique qui sont soumis, en général trois fois par semaine, à une séance d'hémodialyse, d'hémofiltration ou d'hémodiafiltration.

Pour mémoire, l'hémodialyse consiste à faire circuler, de part et d'autre de la membrane semi-perméable d'un hémodialyseur, le sang d'un patient et un liquide de traitement sensiblement isotonique au sang, de sorte que, lors du transfert diffusif qui s'établit au travers de la membrane pour les substances ayant des concentrations différentes de part et d'autre de la membrane, les impuretés du sang (urée, créatinine, etc.) migrent du sang vers le liquide de traitement. La concentration électrolytique du liquide de traitement est aussi généralement choisie pour corriger la concentration électrolytique du sang du patient.

L'hémofiltration consiste à extraire du sang, par ultrafiltration au travers de la membrane semi-perméable d'un échangeur, un volume déterminé d'eau plasmatique chargé en impuretés (filtrat). Ce transfert convectif est provoqué par une différence de pression créée de part et d'autre de la membrane de l'échangeur. Le filtrat est compensé en partie par un liquide de substitution perfusé au patient.

L'hémodiafiltration est une combinaison des deux traitements dont le principe vient d'être rappelé.

Pour évaluer l'efficacité d'une séance de traitement de ce type, c'est-à-dire aussi pour vérifier l'adéquation de la prescription (composition électrolytique du liquide de traitement, débit d'ultrafiltration, durée de la séance, etc.) aux besoins spécifiques de chaque patient, il est du plus haut intérêt de pouvoir mesurer la quantité d'impuretés extraites du sang au cours de la séance de traitement. En particulier, la connaissance de la quantité d'urée éliminée au cours d'une séance permet de calculer le rendement épuratif de l'échangeur utilisé (clairance réelle K pour l'urée) et la dose de dialyse administrée KT/V par la résolution de l'équation C_{UR(aft)} = C_{UR(bef)} x e- KT/V dans laquelle C_{UR(aft)} est la concentration de l'urée dans le sang après traitement, C_{UR(bef)} est la concentration de l'urée dans le sang avant traitement, K est la clairance réelle pour l'urée, T est la durée du traitement et V est le volume d'eau total du patient (Gotch FA, Sargent S.A. A mechanistic analysis of the National Cooperative Dialysis Study (NCDS). Kidney int. 1985 ; 28 ; 526-34).

La mesure de la quantité d'impuretés éliminée au cours d'une séance de traitement, de la quantité d'urée en particulier, pose un problème pratique compte tenu du volume de liquide usé qui est produit, environ cent vingt litres lors d'une séance d'hémodialyse. Il est en effet exclu, notamment pour des raisons sanitaires, de stocker un tel volume de liquide en vue de procéder à l'analyse de son contenu à l'issue de la séance de traitement.

Le document EP 0 621 046 décrit un dispositif permettant de résoudre ce problème. Selon ce document, un dispositif pour recueillir un échantillon de liquide usé représentatif de la totalité du liquide usé évacué au cours d'une séance de traitement comporte :
- une canalisation de prélèvement de liquide usé ayant une première extrémité connectée à une canalisation d'évacuation de liquide usé d'un appareil de traitement de sang par circulation extracorporelle et une seconde extrémité connectable à un récipient de recueil ;
- un débitmètre disposé sur la canalisation d'évacuation de liquide usé en amont de la connection de la canalisation de prélèvement à la canalisation d'évacuation ; et
- une pompe pour provoquer un écoulement dosé de liquide usé dans le récipient de recueil en fonction des informations fournies par le débitmètre.

Il est connu que les pompes sont des moyens de dosages dont la précision n'excède pas, en particulier aux faibles débits, 5 à 10 % selon le type de pompe utilisée. Cette absence de précision a naturellement un retentissement sur la représentativité de l'échantillon recueilli. Par ailleurs, le mode de fonctionnement de certains types d'appareil de traitement extracorporel du sang, ainsi que certains types de traitement altèrent encore cette représentativité.

Un but de l'invention est de réaliser un dispositif de prélèvement d'échantillon de liquide usé qui soit à la fois précis, simple et fiable, quels que soient le type de traitement prescrit et le mode de fonctionnement de l'appareil de traitement de sang utilisé.
Pour atteindre ce but, on prévoit, conformément à l'invention, un dispositif pour recueillir un échantillon de liquide usé représentatif de la totalité du liquide usé évacué à tout moment à partir du début d'une séance de traitement effectuée au moyen d'un appareil de traitement de sang par hémodialyse et/ou hémofiltration comprenant une canalisation d'alimentation en liquide de traitement frais connectable à une entrée d'un hémodialyseur/hémofiltre et une canalisation principale d'évacuation de liquide usé connectable à une sortie d'un hémodialyseur/hémofiltre, ce dispositif comprenant :
- une canalisation de prélèvement de liquide usé ayant une première extrémité connectée à la canalisation principale d'évacuation de liquide usé et une seconde extrémité connectable à un récipient de recueil,
- des premiers moyens pour mesurer une quantité de liquide disposés sur la canalisation d'évacuation principale en amont de la connection de la canalisation de prélèvement à la canalisation d'évacuation principale,
- des moyens pour provoquer un écoulement dosé de liquide usé dans le récipient de recueil en fonction des informations fournies par les premiers moyens de mesure comprenant
   - des seconds moyens de mesure pour mesurer un quantité de liquide, disposés sur la canalisation de prélèvement,
   - des moyens d'obturation, disposés sur la canalisation de prélèvement, et
   - des moyens de commande pour commander, lors d'un traitement par dialyse, l'ouverture des moyens d'obturation chaque fois que les premiers moyens de mesure ont mesuré une première quantité déterminée V de liquide usé et pour commander la fermeture des moyens d'obturation chaque fois que les seconds moyens de mesure ont mesuré une seconde quantité déterminée v de liquide usé.

Selon un mode de réalisation de l'invention, le dispositif est prévu pour coopérer avec un appareil de traitement de sang par hémodialyse et/ou hémofiltration comprenant une canalisation secondaire d'évacuation de liquide usé connectée, en amont des premiers moyens de mesure, à la canalisation d'évacuation principale, cette canalisation étant munie d'une pompe d'extraction et de troisième moyens de mesure pour mesurer une quantité de liquide. Le dispositif comprend alors en outre une canalisation de prélèvement de liquide usé secondaire, ayant une première extrémité connectée à la canalisation d'évacuation secondaire, en aval des troisièmes moyens de mesure, et une seconde extrémité connectable au récipient de recueil. La canalisation d'évacuation secondaire est munie de moyens d'obturation et de quatrième moyens de mesure d'une quantité de liquide. Les moyens de commande sont prévus en outre pour commander, lors d'un traitement par hémofiltration, l'ouverture des moyens d'obturation de la canalisation de prélèvement secondaire chaque fois que les troisièmes moyens de mesure ont mesuré une première quantité déterminée V' de liquide usé et pour commander la fermeture de ces moyens d'obturation chaque fois que les quatrièmes moyens de mesure ont mesuré une seconde quantité déterminée v' de liquide usé. Avantageusement, les deuxième et quatrième moyens de mesure sont confondus et sont disposés sur une portion de canalisation commune aux deux canalisations de prélèvement.

Grâce à cette disposition, si le médecin décide, au cours d'une séance de dialyse, de soumettre le patient pendant une période de temps appréciable, à une séquence d'hémofiltration pure, la représentativité de l'échantillon recueilli n'est pas altérée.

Selon un autre mode de réalisation de l'invention, le dispositif est prévu pour coopérer avec un appareil de traitement de sang par hémodialyse et/ou hémofiltration comprenant des moyens pour permettre, lors de phases d'étalonnage, la circulation de liquide de traitement frais dans une portion. de la canalisation d'évacuation principale comprenant les premiers moyens de mesure. Les moyens de commande sont prévus alors en outre pour commander, lors des phases d'étalonnage, la fermeture des moyens d'obturation de la canalisation de prélèvement.

Dans les appareils qui comportent des organes de mesure similaires (débitmètres, sondes de conductivité, etc.) disposés dans le circuit de liquide de dialyse en amont et en aval de l'échangeur, il est courant, à des fins d'étalonnage, de dériver l'échangeur à intervalles de temps réguliers, de façon à faire circuler dans l'organe de mesure amont comme dans l'organe de mesure aval du liquide de dialyse frais.

Grâce au mode de réalisation qui vient d'être mentionné, le dispositif ne prélève pas de liquide de traitement frais pendant les périodes d'étalonnage.

Selon une caractéristique de l'invention, le dispositif comporte des moyens de régulation du débit dans la canalisation de prélèvement de liquide usé connectée à la canalisation d'évacuation principale

D'autres caractéristiques et avantages de l'invention apparaîtront à la lecture de la description qui va suivre. On se reportera aux dessins annexés sur lesquels :
la figure 1 est un schéma simplifié d'un appareil de traitement de sang par circulation extracorporelle comprenant un premier mode de réalisation de l'invention ; et
la figure 2 est un schéma simplifié d'un appareil de traitement de sang par circulation extracorporelle comprenant un second mode de réalisation de l'invention.

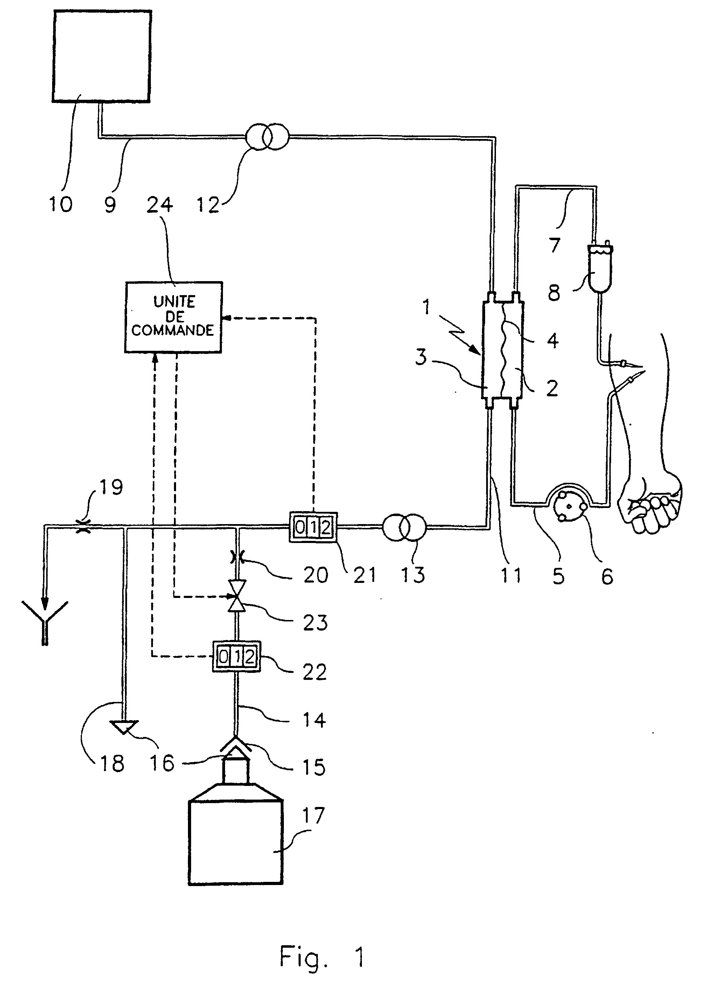

L'appareil de traitement de sang représenté sur la figure 1 comporte un échangeur 1 ayant un premier et un second compartiments 2, 3 séparés par une membrane semi-perméable 4 et reliés respectivement à un circuit pour circulation extracorporelle de sang et à un circuit de liquide de dialyse.

Le circuit pour circulation extracorporelle de sang comprend une canalisation de prélèvement 5 connectée à une entrée du premier compartiment 2, et une canalisation de restitution 7 connectée à une sortie du premier compartiment. Une pompe de circulation 6 est disposée sur la canalisation de prélèvement 5, et un piège à bulle 8 est interposé sur la canalisation de restitution 7.

Le circuit de liquide de dialyse comprend une canalisation d'alimentation 9 en liquide de dialyse frais ayant une extrémité connectée à un générateur de liquide de dialyse 10 et une autre extrémité connectée à une entrée du second compartiment 3, et une canalisation d'évacuation 11 ayant une extrémité connectée à une sortie du second compartiment 3 et une extrémité reliée à l'égout.

Le circuit de liquide de dialyse est muni de deux pompes de circulation 12, 13 disposées respectivement en amont et en aval de l'échangeur 1, dont les débits sont commandés pour que le débit de l'eau plasmatique ultrafiltrant au travers de la membrane 4 soit égal à un débit souhaité.

Conformément à l'invention l'appareil de traitement qui vient d'être décrit est muni d'un dispositif pour recueillir un échantillon de liquide usé représentatif, à tout moment, de la totalité du liquide usé évacué depuis le début de la séance de traitement.

Ce dispositif comprend une canalisation de prélèvement 14 ayant une extrémité connectée à la canalisation d'évacuation 11 et une autre extrémité munie d'un raccord 15 apte à coopérer avec un raccord complémentaire 16 d'un conteneur de recueil 17. Pour permettre le nettoyage et la désinfection de la canalisation de prélèvement 14 durant les cycles d'entretien de l'appareil de traitement, une canalisation de bouclage 18 est connectée à la canalisation d'évacuation 11, dont l'extrémité libre est munie d'un raccord 16 complémentaire du raccord 15. Les canalisations d'évacuation 11 et de prélèvement 14 sont munies chacune d'un organe de rétreint 19, 20 respectivement disposé en aval de leur jonction. Les organes de rétreint 19, 20 sont calibrés de façon que le rapport des débits des fluides circulant dans les deux canalisations corresponde approximativement à une fraction du volume total de liquide à échantillonner (dix pour cent par exemple) qui soit aussi un multiple du débit d'échantillonnage. L'organe de rétreint 19 disposé sur la canalisation d'évacuation 11 est de préférence constitué par un accessoire de l'appareil de traitement remplissant une autre fonction spécifique, tel qu'un échangeur de température. L'organe de rétreint 20 disposé sur la canalisation de prélèvement 14 peut être constitué par une portion de canalisation ayant un diamètre calibré.

Le dispositif d'échantillonnage selon l'invention comprend en outre des premiers moyens de mesure d'une quantité de liquide, tels qu'un compteur volumétrique 21, disposés sur la canalisation d'évacuation 11 en amont de sa jonction à la canalisation de prélèvement 14, des seconds moyens de mesure d'une quantité de liquide, tels qu'un compteur volumétrique de liquide 22, disposés sur la canalisation de prélèvement 14, et des moyens d'obturation, tels qu'une vanne 23, disposés aussi sur la canalisation de prélèvement 14. Une unité de commande 24 reçoit les signaux émis par les deux compteurs volumétriques 21, 22 et commande l'ouverture de la vanne 23 selon une séquence telle que le volume de liquide recueilli dans le récipient 17 soit égal, à tout moment, à une fraction déterminée (un pour cent, par exemple) du volume total du liquide usé qui s'est écoulé dans la canalisation d'évacuation 11 depuis le début de la séance.

Le fonctionnement de cet appareil est le suivant : Après que le circuit de circulation extracorporelle de sang et le circuit de liquide de dialyse ont été connectés à l'échangeur 1, puis rincés et remplis, les canalisations de prélèvement 5 et de restitution 7 de sang sont connectées au circuit vasculaire d'un patient.

Un récipient 17 de recueil d'échantillon de liquide usé vide est relié à la ligne de prélèvement d'échantillon 14 au moyen des raccords 15, 16.

La pompe 6 de circulation de sang est alors mise en service, ainsi que les pompes 12, 13 du circuit de dialyse, dont les débits sont ajustés pour que le second compartiment 3 de l'échangeur 1 soit le siège d'une dépression par rapport au premier compartiment 2 et que, en conséquence, une fraction prédéterminée d'eau plasmatique migre du circuit sang vers le circuit de liquide de dialyse. L'épuration par transfert convectif et diffusif commence dans l'échangeur.

Dès que le liquide de rinçage contenu dans la canalisation d'évacuation 11 de liquide usé a été purgé, le dispositif de prise d'échantillon selon l'invention est mis en service. Chaque fois que le premier compteur volumétrique 21 a mesuré une quantité déterminée V de liquide, l'unité de commande 24 provoque l'ouverture de la vanne 23 de sorte que du liquide usé s'écoule dans le récipient de recueil 17. Dès que le second compteur volumétrique 22 a mesuré une quantité déterminée v de liquide, l'unité de commande 24 provoque la fermeture de la vanne 23. Et ainsi de suite, jusqu'à la fin de la séance de traitement.

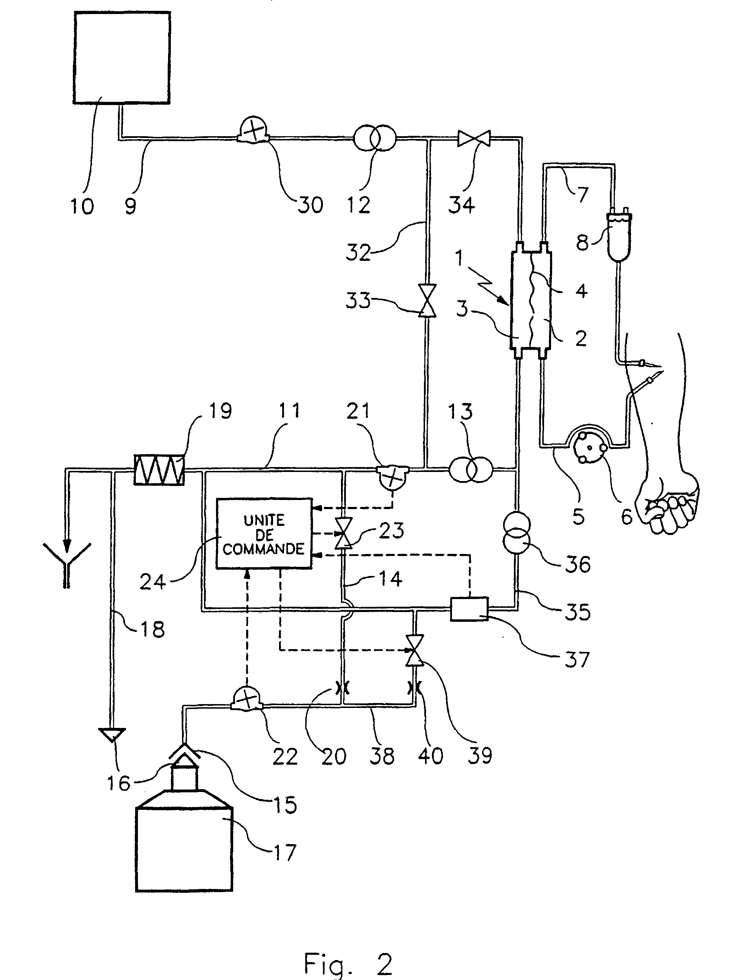

Le dispositif de traitement représenté sur la figure 2 se différencie de celui qui est représenté sur la figure 1 en ce qu'il comprend un dispositif de mesure du débit d'ultrafiltration dans l'échangeur nécessitant de faire circuler, à intervalles de temps réguliers, du liquide de dialyse frais dans la canalisation de liquide usé. Le dispositif de recueil d'échantillon de liquide usé de cet appareil est adapté pour tenir compte de cette contrainte. Il est aussi prévu pour un fonctionnement de l'appareil en mode hémofiltration, pendant lequel le seul liquide qui circule dans le circuit de liquide de dialyse est de l'ultrafiltrat.

Les éléments identiques ou similaires des appareils des figures 1 et 2 sont repérés par les mêmes chiffres.

Le dispositif de régulation du débit d'ultrafiltration de cet appareil de traitement comprend un premier débitmètre 30 disposé sur la canalisation d'alimentation 9, en amont de la première pompe de circulation 12, et un second débitmètre 21 disposé sur la canalisation d'évacuation 11, en aval de la seconde pompe de circulation 13. La pompe 13 est asservie à la comparaison des débits mesurés par les deux débitmètres 30, 21 de façon que ces débits soient égaux. Les débitmètres 30, 21 sont placés directement en série à intervalles de temps réguliers pour une phase d'étalonnage ; à cette fin, une canalisation d'étalonnage 32 est montée en dérivation à l'échangeur 1, entre un point de jonction à la canalisation d'alimentation 9, situé en aval de la première pompe de circulation 12, et un point de jonction à la canalisation d'évacuation 11, situé en aval de la deuxième pompe de circulation 13. La canalisation d'étalonnage 32 est munie d'une vanne 33 et la canalisation d'alimentation 9 est munie d'une vanne 34, la mise en service et hors. service de la canalisation d'étalonnage 32 étant commandée par l'ouverture d'une vanne et la fermeture de l'autre vanne et vice versa.

Une canalisation d'évacuation secondaire 35 est connectée en dérivation à la canalisation d'évacuation 11, entre un point situé en amont de la seconde pompe de circulation 13 et un point situé en amont d'un échangeur de température 19 disposé à proximité de l'extrémité libre de la canalisation d'évacuation 11. Sur cette canalisation d'évacuation secondaire sont disposés, en série, une pompe d'extraction 36 et un troisième dispositif 37 de mesure d'une quantité de liquide, tel que celui qui est décrit par exemple dans le brevet européen no. 0 401 139. Comme les quantités de liquide circulant dans les débitmètres 30, 21 sont maintenues égales, le volume de liquide usé pompé par la pompe d'extraction 36 hors du circuit de liquide de dialyse et refoulé vers l'égout correspond exactement au volume d'eau plasmatique passant par ultrafiltration au travers de la membrane 4 de l'échangeur 1.

Outre les éléments qui ont été décrit plus haut en relation à la figure 1, le dispositif de recueil d'échantillon de l'appareil représenté sur la figure 2 comprend une seconde canalisation 38 de prélèvement de liquide usé ayant une extrémité connectée à la canalisation d'évacuation secondaire 35, en aval des troisièmes moyens 37 de mesure d'une quantité de liquide, et une autre extrémité connectable au récipient de recueil 17. Sur cette seconde canalisation de prélèvement 38 sont disposés des moyens d'obturation, tels qu'une vanne 39, un organe de rétreint 40 et des quatrièmes moyens 22 de mesure d'une quantité de liquide, tels qu'un débitmètre. Avantageusement, comme représenté sur la figure 2, les deuxièmes et quatrièmes moyens de mesure d'une quantité de liquide sont confondus (débitmètre 22) et sont disposés sur une portion de canalisation commune aux deux canalisations de prélèvement 38, 14.

L'appareil de traitement qui vient d'être décrit fonctionne de la manière suivante :

Lors d'un traitement par dialyse (qui comporte en général toujours l'ultrafiltration nécessaire pour faire perdre au patient son excès de poids) de même que lors d'un traitement par hémodiafiltration , la vanne 34 est ouverte, la vanne 33 est fermée, les deux pompes 12 et 13 tournent, la pompe 12 à un régime fixe et la pompe 13 à un régime variable ajusté pour que les débits d'entrée et de sortie de liquide au niveau des deux débitmètres 30, 21 soient égaux. La pompe d'extraction 36, dont le débit est régulé au moyen du dispositif de mesure d'une quantité de liquide 37, tourne pour extraire du circuit de dialyse une quantité de liquide correspondant à une quantité d'ultrafiltrat souhaitée.

L'unité de commande calcule des volumes de liquide successifs V = V1 + V2 , égaux à la somme de volumes V1 mesurés au moyen du débitmètre 21 et de volumes V2 mesurés au moyen du dispositif de mesure 37. Chaque fois qu'une quantité de liquide usé V = V1 + V2 s'est écoulée dans les canalisations d'évacuation 11 et 35, l'unité de commande 24 provoque l'ouverture de la vanne 23 et, par voie de conséquence, l'écoulement de liquide dans le récipient de recueil 17. Chaque fois qu'une quantité de liquide v, calculée par l'unité de commande 24 à partir des informations délivrées par le débitmètre 22 situé sur la canalisation de prélèvement 14, s'est écoulée au travers de ce débitmètre, l'unité de commande 24 provoque la fermeture de la vanne 23.

Au cours de la séance de dialyse, les débitmètres amont et aval 30, 21 sont étalonnés régulièrement. La vanne 34 est alors fermée, la vanne 33 est ouverte et la pompe 13 est stoppée. Le liquide qui circule dans la canalisation d'évacuation 11 est du liquide de dialyse frais. Pour éviter d'échantillonner du liquide de dialyse frais, ce qui ne présente aucun intérêt pratique, la vanne 23 reste fermée entre le moment où les vannes 33, 34 sont positionnées pour permettre l'étalonnage des débitmètres et le moment où leurs positions sont inversées pour rétablir la circulation de liquide de dialyse dans l'échangeur 1.

En mode d'ultrafiltration, seules fonctionnent la pompe d'ultrafiltration 36 et la pompe à sang 6. Tout le liquide usé s'écoule par la canalisation d'évacuation secondaire 35. Chaque fois qu'une quantité V' de liquide a été mesurée par l'organe de mesure 37, l'unité de commande 24 provoque l'ouverture de la vanne 39 et chaque fois qu'une quantité v' de liquide, calculée par l'unité de commande 24 à partir des informations délivrées par le débitmètre 22, s'est écoulée au travers de ce débitmètre, l'unité de commande provoque la fermeture de la vanne 39.

Les rapports V/v et V'/v' sont égaux.

L'invention n'est pas limitée aux modes de réalisation qui viennent d'être décrits et elle est susceptible de variantes. En particulier, il est possible de remplacer les organes de rétreint 19, 20, grâce auxquels le rapport entre le débit total de liquide usé et le débit de liquide d'échantillonné est sensiblement constant, par un régulateur de pression, disposé en aval de la jonction de la canalisation d'évacuation 11 à la canalisation de prélèvement 14, grâce auquel le débit de liquide échantillonné serait sensiblement constant.

## Revendications

1. Dispositif pour recueillir un échantillon de liquide usé représentatif de la totalité du liquide usé évacué à tout moment à partir du début d'une séance de traitement effectuée au moyen d'un appareil de traitement de sang par hémodialyse et/ou hémofiltration comprenant une canalisation (9) d'alimentation en liquide de traitement frais connectable à une entrée d'un hémodialyseur/hémofiltre (1) et une canalisation (11) principale d'évacuation de liquide usé connectable à une sortie d'un hémodialyseur/hémofiltre (1), le dispositif comprenant :
- une canalisation (14) de prélèvement de liquide usé ayant une première extrémité connectée à la canalisation (11) principale d'évacuation de liquide usé et une seconde extrémité connectable à un récipient de recueil (17),
- des premiers moyens de mesure (21) pour mesurer une quantité de liquide disposés sur la canalisation (11) d'évacuation principale en amont de la connection de la canalisation (14) de prélèvement à la, canalisation (11) d'évacuation principale ;
- des moyens (22, 23, 24) pour provoquer un écoulement dosé de liquide usé dans le récipient de recueil (17) en fonction des informations fournies par les premiers moyens de mesure (21) ;
**caractérisé en ce que** les moyens pour provoquer un écoulement dosé de liquide usé comprennent :
- des seconds moyens de mesure (22) pour mesurer une quantité de liquide, disposés sur la canalisation (14) de prélèvement ;
- des moyens d'obturation (23), disposés sur la canalisation de prélèvement (14) ;
- des moyens (24) de commande pour commander, lors d'un traitement par dialyse, l'ouverture des moyens d'obturation (23) chaque fois que les premiers moyens de mesure (21) ont mesuré une première quantité déterminée V de liquide usé et pour commander la fermeture des moyens d'obturation (23) chaque fois que les seconds moyens de mesure (22) ont mesuré une seconde quantité déterminée v de liquide usé.

2. Dispositif selon la revendication 1 pour coopérer avec appareil de traitement de sang par hémodialyse et/ou hémofiltration comprenant une canalisation (35) secondaire d'évacuation de liquide usé connectée, en amont des premiers moyens de mesure (21), à la canalisation (11) d'évacuation principale, cette canalisation étant munie d'une pompe d'extraction (36) et de troisième moyens de mesure (37) pour mesurer une quantité de liquide,
le dispositif étant **caractérisé en ce qu'**il comprend en outre une canalisation (38) de prélèvement de liquide usé secondaire, ayant une première extrémité connectée à la canalisation (35) d'évacuation secondaire, en aval des troisièmes moyens de mesure (37), et une seconde extrémité connectable au récipient (17) de recueil, cette canalisation étant munie de moyens d'obturation (39) et de quatrième moyens de mesure (22) d'une quantité de liquide, les moyens de commande (24) étant prévus en outre pour commander, lors d'un traitement par hémofiltration, l'ouverture des moyens d'obturation (39) de la canalisation (38) de prélèvement secondaire chaque fois que les troisièmes moyens de mesure (37) ont mesuré une première quantité déterminée V' de liquide usé et pour commander la fermeture des moyens d'obturation (39) chaque fois que les quatrièmes moyens de mesure (22) ont mesuré une seconde quantité déterminée v' de liquide usé.

3. Dispositif selon la revendication 2, **caractérisé en ce que** les deuxième et quatrième moyens de mesure sont confondus et sont disposés sur une portion de canalisation commune aux deux canalisations (14, 38) de prélèvement de liquide usé.

4. Dispositif selon une des revendications 1 à 3, pour coopérer avec un appareil de traitement de sang par hémodialyse et/ou hémofiltration comprenant des moyens (32, 33, 34) pour permettre, lors de phases d'étalonnage, la circulation de liquide de traitement frais dans une portion de la canalisation (11) d'évacuation principale comprenant les premiers moyens de mesure (21),
le dispositif étant **caractérisé en ce que** les moyens de commande (24) sont prévus en outre pour commander, lors des phases d'étalonnage, la fermeture des moyens d'obturation (23) de la canalisation (14) de prélèvement de liquide usé.

5. Dispositif selon une des revendications 1 à 4, **caractérisé en ce qu'**il comporte des moyens de régulation (19, 20) du débit dans la canalisation (14) de prélèvement de liquide usé connectée à la canalisation (11) d'évacuation principale.

6. Dispositif selon la revendications 5, **caractérisé en ce que** les moyens de régulation du débit dans la canalisation (14) de prélèvement de liquide usé comportent un premier organe (20) de restriction d'écoulement de liquide disposé sur la canalisation (14) de prélèvement et un second organe (19) de restriction d'écoulement de liquide disposé sur la canalisation (11) d'évacuation principale en aval de la jonction de cette canalisation (11) à la canalisation (14) de prélèvement, de sorte que le débit dans la canalisation (14) de prélèvement est sensiblement égal à une fraction déterminée du débit dans la canalisation (11) d'évacuation.

7. Dispositif selon la revendications 5, **caractérisé en ce que** les, moyens de régulation du débit dans la canalisation de prélèvement de liquide usé comportent un régulateur de pression disposé sur la canalisation d'évacuation principale en aval de la jonction de cette canalisation à la canalisation de prélèvement, de sorte que le débit dans la canalisation de prélèvement est sensiblement constant.

8. Dispositif selon une des revendication 1 à 7, **caractérisé en ce que** les premiers moyens de mesure (21) comprennent un compteur volumétrique.

9. Dispositif selon une des revendications 1 à 8, **caractérisé en ce que** les seconds moyens de mesure (22) comprennent un compteur volumétrique.

10. Dispositif selon une des revendications 1 à 8, caractérisé en ce les moyens d'obturation disposés sur l'une et/ou l'autre canalisation (14, 38) de prélèvement de liquide usé comprennent un vanne (23, 39) .

## Patentansprüche

1. Vorrichtung zur Sammlung einer Probe gebrauchter Flüssigkeit, die für die Gesamtheit der gebrauchten Flüssigkeit repräsentativ ist, die seit dem Beginn einer Behandlungssitzung abgeführt wird, die mittels einer Blutbehandlungsvorrichtung mit Hämodialyse und/oder Hämofiltration durchgeführt wird, mit einer Leitung (9) zur Versorgung mit frischer Behandlungsflüssigkeit, die an einen Eingang eines Hämodialysators/Hämofilters (1) anschließbar ist, und einer Hauptleitung (11) für den Abfluss von gebrauchter Flüssigkeit, die an einen Ausgang eines Hämodialysators/Hämofilters (1) anschließbar ist; wobei die Vorrichtung umfasst:
- eine Leitung (14) zur Entnahme gebrauchter Flüssigkeit mit einem ersten Ende, das an die Hauptabflussleitung (11) gebrauchter Flüssigkeit angeschlossen ist, und einem zweiten Ende, das an einen Sammelbehälter (17) anschließbar ist,
- erste Messmittel (21) zum Messen einer Flüssigkeitsmenge, die in der Hauptabflussleitung (11) stromaufwärts von der Verbindung der Entnahmeleitung (14) mit der Hauptabflussleitung (11) angeschlossen ist,
- Mittel (22, 23, 24) zum Auslösen eines dosierten Abflusses von gebrauchter Flüssigkeit in den Sammelbehälter (17) in Abhängigkeit von Informationen, die von den ersten Messmitteln (21) geliefert werden,
**dadurch gekennzeichnet, dass** die Mitteln zum Auslösen eines dosierten Abflusses von gebrauchter Flüssigkeit folgendes umfassen:
- zweite Messmittel (22) zum Messen einer Flüssigkeitsmenge, die in der Entnahmeleitung (14) angeordnet sind,
- Verschlussmittel (23), die in der Entnahmeleitung (14) angeordnet sind,
- Steuermittel (24), um bei einer Dialysebehandlung das Öffnen der Verschlussmittel (23) jedes Mal dann zu bewirken, wenn die ersten Messmittel (21) eine erste bestimmte Menge V gebrauchter Flüssigkeit gemessen haben, und um das Schließen der Verschlussmittel (23) jedes Mal dann zu bewirken, wenn die zweiten Messmittel (22) eine zweite bestimmte Menge v gebrauchter Flüssigkeit gemessen haben.

2. Vorrichtung nach Anspruch 1 für das Zusammenwirken mit einer Blutbehandlungsvorrichtung durch Hämodialyse und/oder Hämofiltration mit einer sekundären Leitung (35) für den Abfluss gebrauchter Flüssigkeit, die stromaufwärts von den ersten Messmitteln (21) an die Hauptabflussleitung (11) angeschlossen sind, wobei diese Leitung mit einer Entnahmepumpe (36) und dritten Messmitteln (37) zum Messen einer Flüssigkeitsmenge versehen ist,
**dadurch gekennzeichnet, dass** sie zudem eine sekundäre Leitung (38) zur Entnahme gebrauchter Flüssigkeit umfasst, die ein erstes Ende aufweist, das stromabwärts von den dritten Messmitteln (37) an die sekundäre Abflussleitung (35) angeschlossen ist, und ein zweites Ende, das an den Sammelbehälter (17) anschließbar ist, wobei diese Leitung mit Verschlussmitteln (39) und vierten Messmitteln (22) für eine Flüssigkeitsmenge versehen ist, wobei die Steuermittel (24) außerdem dafür vorgesehen sind, bei einer Behandlung durch Hämofiltration die Öffnung der Verschlussmittel (39) der sekundären Entnahmeleitung (38) jedes Mal dann zu bewirken, wenn die dritten Messmittel (37) eine erste bestimmte Menge V' gebrauchter Flüssigkeit gemessen haben, und um das Schließen der Verschlussmittel (39) jedes Mal dann zu bewirken, wenn die vierten Messmittel (22) eine zweite bestimmte Menge v' gebrauchter Flüssigkeit gemessen haben.

3. Vorrichtung nach Anspruch 2, **dadurch gekennzeichnet, dass** die zweiten und vierten Messmittel verschmolzen und in einem Leitungsteil angeordnet sind, der den zwei Leitungen (14, 38) zur Entnahme gebrauchter Flüssigkeit gemeinsam ist.

4. Vorrichtung nach einem der Ansprüche 1 bis 3 für das Zusammenwirken mit einer Blutbehandlungsvorrichtung durch Hämodialyse und/oder Hämofiltration mit Mitteln (32, 33, 34), um während Eichungsphasen die Zirkulation von frischer Behandlungsflüssigkeit in einem Teil der Hauptabflussleitung (11) zu gestatten, die die ersten Messmittel (21) umfasst,
**dadurch gekennzeichnet, dass** die Steuermittel (24) außerdem dafür vorgesehen sind, bei den Eichungsphasen die Schließung der Verschlussmittel (23) der Entnahmeleitung (14) für gebrauchte Flüssigkeit zu bewirken.

5. Vorrichtung nach einem der Ansprüche 1 bis 4, **dadurch gekennzeichnet, dass** sie Regelmittel (19, 20) für den Durchfluss in der Leitung (14) zur Entnahme gebrauchter Flüssigkeit umfasst, die an die Hauptabflussleitung (11) angeschlossen ist.

6. Vorrichtung nach Anspruch 5, **dadurch gekennzeichnet, dass** die Regelmittel für den Durchfluss in der Leitung (14) zur Entnahme gebrauchter Flüssigkeit ein erstes Organ (20) zur Begrenzung des Flüssigkeitsstroms, das in der Entnahmeleitung (14) angeordnet ist, und ein zweites Organ (19) zur Begrenzung des Flüssigkeitsstroms umfasst, das in der Hauptabflussleitung (11) stromabwärts von der Verbindung dieser Leitung (11) mit der Entnahmeleitung (14) angeordnet ist, so dass der Durchfluss in der Entnahmeleitung (14) im Wesentlichen gleich einem bestimmten Bruchteil des Durchflusses in der Abflussleitung (11) ist.

7. Vorrichtung nach Anspruch 5, **dadurch gekennzeichnet, dass** die Regelmittel für den Durchfluss in der Leitung zur Entnahme gebrauchter Flüssigkeit einen Druckregler umfassen, der in der Hauptabflussleitung stromabwärts von der Verbindung dieser Leitung mit der Entnahmeleitung angeordnet ist, so dass der Durchfluss in der Entnahmeleitung im Wesentlichen konstant ist.

8. Vorrichtung nach einem der Ansprüche 1 bis 7, **dadurch gekennzeichnet, dass** die ersten Messmittel (21) einen Volumenzähler umfassen.

9. Vorrichtung nach einem der Ansprüche 1 bis 8, **dadurch gekennzeichnet, dass** die zweiten Messmittel (22) einen Volumenzähler umfassen.

10. Vorrichtung nach einem der Ansprüche 1 bis 8, **dadurch gekennzeichnet, dass** die Verschlussmittel, die an der einen und/oder der anderen Leitung (14, 38) zur Entnahme gebrauchter Flüssigkeit angeordnet sind, ein Ventil (23, 39) umfassen.

## Claims

1. Device for collecting a waste liquid sample representative of all the waste liquid discharged at any time from the beginning of a treatment session carried out by means of an apparatus for treatment of blood by haemodialysis and/or haemofiltration comprising a feedline (9) for fresh treatment liquid which can be connected to an inlet of a haemodialyser/haemofilter (1) and a main waste liquid discharge line (11) which can be connected to an outlet of a haemodialyser/haemofilter (1), the device comprising:
- a waste liquid withdrawal line (14) having a first end connected to the main waste liquid discharge line (11) and a second end which can be connected to a collecting container (17),
- first measurement means (21) for measuring an amount of liquid arranged on the main discharge line (11) upstream of the connection of the withdrawal line (14) to the main discharge line (11),
- means (22, 23, 24) for causing a metered flow of waste liquid into the collecting container (17) as a function of the data supplied by the first measurement means (21),
**characterized in that** the means for causing a metered flow of waste liquid comprise:
- second measurement means (22) for measuring an amount of liquid arranged on the withdrawal line (14),
- obturation means (23) arranged on the withdrawal line (14),
- control means (24) for controlling, during a treatment by dialysis, the opening of the obturation means (23) each time that the first measurement means (21) have measured a first predetermined amount V of waste liquid and for controlling the closing of the obturation means (23) each time that the second measurement means (22) have measured a second predetermined amount v of waste liquid.

2. Device according to Claim 1 for interacting with an apparatus for treatment of blood by haemodialysis and/or haemofiltration comprising a secondary waste liquid discharge line (35) connected, upstream of the first measurement means (21), to the main discharge line (11), this line being equipped with an extraction pump (36) and with third measurement means (37) for measuring an amount of liquid,
the device being **characterized in that** it additionally comprises a secondary waste liquid withdrawal line (38) having a first end connected to the secondary discharge line (35), downstream of the third measurement means (37), and a second end which can be connected to the collecting container (17), this line being equipped with obturation means (39) and with fourth means (22) for measuring an amount of liquid, the control means (24) being designed in addition to control, during a treatment by haemofiltration, the opening of the means for obturation (39) of the secondary withdrawal line (38) each time that the third measurement means (37) have measured a first predetermined amount V' of waste liquid and to control the closing of the obturation means (39) each time that the fourth measurement means (22) have measured a second predetermined amount v' of waste liquid.

3. Device according to Claim 2, **characterized in that** the second and fourth measurement means are coincident and are arranged on a portion of line common to the two waste liquid withdrawal lines (14, 38).

4. Device according to one of Claims 1 to 3, for interacting with an apparatus for treatment of blood by haemodialysis and/or haemofiltration comprising means (32, 33, 34) for making possible, during calibration phases, the circulation of fresh treatment liquid in a portion of the main discharge line (11) comprising the first measurement means (21),
the device being **characterized in that** the control means (24) are additionally designed to control, during calibration phases, the closing of the means (23) for obturation of the waste liquid withdrawal line (14).

5. Device according to one of Claims 1 to 4, **characterized in that** it contains means (19, 20) for adjusting the flow rate in the waste liquid withdrawal line (14) connected to the main discharge line (11).

6. Device according to Claim 5, **characterized in that** the means for adjusting the flow rate in the waste liquid withdrawal line (14) include a first liquid flow restriction element (20) arranged on the withdrawal line (14) and a second liquid flow restriction element (19) arranged on the main discharge line (11) downstream of the junction of this line (11) with the withdrawal line (14), so that the flow rate in the withdrawal line (14) is substantially the same as a predetermined fraction of the flow rate in the discharge line (11).

7. Device according to Claim 5, **characterized in that** the means for adjusting the flow rate in the waste liquid withdrawal line comprise a pressure regulator arranged on the main discharge line downstream of the junction of this line with the withdrawal line, so that the flow rate in the withdrawal line is substantially constant.

8. Device according to one of Claims 1 to 7, **characterized in that** the first measurement means (21) comprise a volumetric meter.

9. Device according to one of Claims 1 to 8, **characterized in that** the second measurement means (22) comprise a volumetric meter.

10. Device according to one of Claims 1 to 8, **characterized in that** the obturation means arranged on one and/or the other waste liquid withdrawal line (14, 38) comprise a valve (23, 39).
